Europäisches Patentamt

European Patent Office

Office européen des brevets

(11) Publication number: **0 396 117**
**A2**

## (12) EUROPEAN PATENT APPLICATION

(21) Application number: **90108323.8**

(51) Int. Cl.⁵: **A61L 2/18**

(22) Date of filing: **02.05.90**

(30) Priority: **03.05.89 IT 8253689**

(43) Date of publication of application:
**07.11.90 Bulletin 90/45**

(84) Designated Contracting States:
**AT BE CH DE DK ES FR GB GR IT LI LU NL SE**

(71) Applicant: **KEOMA SRL**
**Zona Ind., le Sant'Andrea**
**I-34170 Gorizia(IT)**

(72) Inventor: **Toson, Carlo**
**Via Crimea 32**
**I-Percoto, Udine(IT)**

(74) Representative: **Tiedtke, Harro, Dipl.-Ing. et al**
**Patentanwaltsbüro Tiedtke-Bühling-Kinne-**
**Grupe-Pellmann-Grams-Struif Bavariaring 4**
**Postfach 20 24 03**
**D-8000 München 2(DE)**

(54) **Process and plant for the disinfection of sanitary fittings, particularly for hydromassage.**

(57) For the disinfection and the cleaning of hydromassage installations the components are filled with disinfecting solution, apart from the bath tub, the solution being circulated under reduced pressure and with the delivery fittings in the closed condition, the rinsing being thereafter carried out in the same manner.

Fig.1

EP 0 396 117 A2

The present invention relates to the disinfection of sanitary installations, particularly for hydromassage.

It is known that in the sanitary plants, particularly for hydromassage, the requirement exists of a disinfection, this requirement being particularly important when the installation is not used within a strictly family environnment, such as in hotels, public sanitary services (also known as diurnal hotels) athetic clubs and the like.

On the other side the simplest solution, which at the same time gives the necessary safety of disinfection level, namely of contact and permanency of the disinfecting liquid not only with the walls of the bath tub or shower cabin, but also with the pipes, fittings etc. forming the installation, this solution consisting in the filling the bath tub and having it carrying out an even shortened operating cycle, is objectionable and disadvantageous under two point of view.

Firstly a relevant amount of disinfecting agent is consumed, owing to the not negligeable volume of liquid in the cycle, and secondly the time needed for the filling, emptying and disinfection operation (shortened cycle) is not consistant with the desirable frequency of installation use.

In recent years other solutions have been proposed which appeared to be complicated and most of times less effective than the above described one.

As a matter of fact, the main problem to date faced is that of assuring an effective disinfection of the components of the plant which are not easily accessible: otherwise stated the cleaning and the disinfection of the bath tub are carried out readily and easily, whereas that of the pipes, fittings and pump are satisfactorily carried out only when the bath tub is filled with water containing the disinfecting agent and the plant is operated for a shortened time but sufficient to ensure an effective disinfection.

In the following description reference shall be made to a hydromassage plant, it being meant that the invention may find use as a general prin ciple in other tipes of sanitary plants, with the evident and suitable modifications.

The main purpose of the present invention is that of providing a plant and a process for the disinfection and cleaning of the components of the hydromassage installation, capable of assuring such a disinfection with a very reduced consumption of disinfecting agents and in a very short times.

Another purpose of the invention is that of providing a disinfection and cleaning plant of the above mentioned type so that the plant has a minimum encumbrance and is readily integrated both with the sanitary installation with which it is combined and with aesthetical and functional requisites of other components such as the bath tub.

These and other purposes are achieved by means of the disinfection process according to the present invention which is characterized by the steps of filling the pipes of the hydromassage installation, including the suction pipe and the delivery pipe of the circulation pump, with a 2% aqueous solution of disinfecting agent and then operating the circulation pump so as to generate a reduced hydraulic pressure, this operation being carried out for about 2-3 minutes, the disinfecting solution being then discharged and a like rincing cycle being carried out with clean water. In turn the plant according to the invention is characterized, in its most general embodiment, by a feeding assembly for disinfecting aqueous solution having a predetermined concentration to the hydromassage installation, preferably at the suction pipe of the circulation pump, in an amount sufficient to fill all the components of the installation apart from the bath tub and by control means of the operation cycle and of the operation rate of the circulation pump so that the hydraulic pressure originated during the operation of the circulation pump is less than a predetermined minimum value.

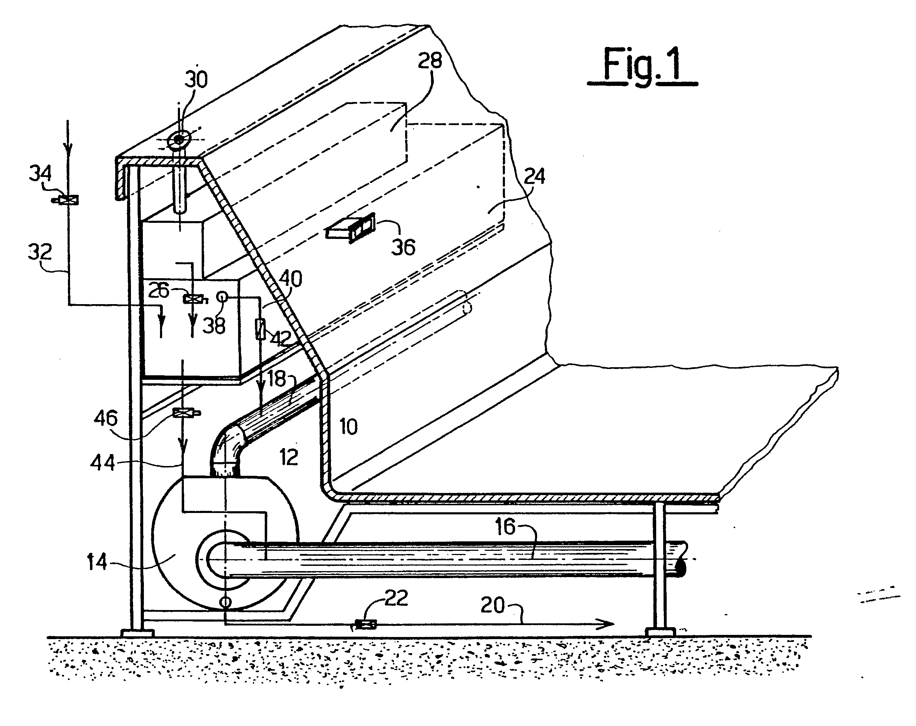

According to the preferred embodiment of the plant of the present invention said feeding assembly for the disinfecting solution comprises a first tank for mixing water and disinfecting agent and a second tank of disinfecting agent stock connected to said first tank by means for the supply of a dosed amount , said first tank having a filling value corresponding to that of the plant components to be initially filled in. A particular features and advantages of the present invention shall be better understood from the following detailed description, made with respect to the enclosed drawing (figure 1) which shows in a schematic form the plant according to a preferred embodiment thereof , it having no limiting meaning.

In the drawing the reference 10 show the wall of a hydromassage bath tub defining a cavity 12 in which the conventional components of a hydromassage installation, namely the pump 14, the related suction pipe 16 and the delivered pipe 18, as well the discharge pipe 20 having a discharge electric valve 22, are housed.

Of course the delivery pipe 18 suppplies the fittings (not shown) distributed along the bath tub, whereas the suctions pipe 16 is connected to a suction fitting (also not shown) provided in the bath tub, besides the normal discharge fitting.

It is to be observed that the hydromassage fittings are conventionally provided with a manual closing system, to prevent the jet of water mixed with air coming out from them.

In more recent years fittings have been described in which the aforesaid closure is obtained by reducing the hydraulic pressure feeding the fitting themselves.

Plants exist in which the aforesaid suction fitting is combined with the conventional discharge fitting of the bath tub.

Moreover at the same time as the present application is filed, a copending patent application is filed relating to an improved fitting in which the hydraulic pressure delivered by the circulation pump controls the opening of the fitting which is moreover provided with an internal outlet, which can be opened and closed, connected to a collecting headers which in turn is connected to the suction pipe of the circulation pump. In the preferred embodiment of the present invention reference is made to a hydromassage installation, having fittings of the latter type, whereby the operation of the pump under reduced rate causes the delivery of a pressure not sufficient to have the fittings opened, whereby the water (in this case disinfecting solution) fed to the fittings is recirculated through the outlet of the fittings, the aforesaid header and the circulation pump.

Coming back to the figure 1, the plant according to the invention, comprises a tank 24 for mixing water and disinfecting agent, the latter being fed in doses amount through the charging electrovalve 26 from a reserve tank 28, containing a predetermined number of doses for a like number of disinfection cycles.

The disinfecting agents are per se known, (normally capable of the developing oxygen of chlorine and for the purposes of the present invention are preferably liquids.

A filler 30 permits the reserve tank 28 to be replenished.

The tank 24 is fed with tap water through the line 32 having a control electrovalve 34.

A control switch 36 permits the amount of liquid or load of the tank 24 to be adjusted whereas an overflow 38 discharges the tank content into the delivery pipe 18 through the line 40 and the electrovalve 42.

The ordinary outlet of the tank 24 comprises the pipe 44, having the control electrovalve 46, connected to the suction pipes 16 of the pump 14.

In the case the switch 36 or the electrovalve 34 should not correctly operate to stop the inlet of water within the tank 24 the overflow 38 (together with the line 40 and the valve 42) takes care of the emergency discharge.

The operation of the above described plant shall be now described with reference to the process of the present invention.

It is firstly to be noted that, specifically with reference to the use in hydromassage plants, the operation contemplates two modes, namely either manual and/or automatic.

In the first case suitable control knobs shall be used, positioned onto an auxilliary panel, whereas in the second case the disinfection cycle shall autmatically begin each time a hydromassage cycle is ended, this end being signalled by the complete discharge of the water contained in the bath tub. It is evident that suitable consent device may be used confirming that a hydromassage cycle took effectively place (such as for example the fact the in the bath tub water has been loaded up to above a minimum level and that the hydromassage fittings or the circulation pump have been actuated for a time greater than a predertermined minimum value).

By opening the valve 26 the predetermined dosed amount of disinfecting agent, taken from tile tank 23 , is loaded within the tank 24.

Upon the valve 26 is closed, the valve 34 is opened, thus feeding within the tank 24 the necessary water amount has indicated by the control switch 36.

At this point, once the valve 34 is closed, the valve 46 is opened whereby the full content of the tank 24 is discharged within the suction pipe of the pump 14 , and consequently all the pipes of the plant , including the fittings, are filled. To this end the plant shall be provided with one or more venting device for the air contained in the pipes themselves.

When the filling is completed and the valve 46 is closed, the circulation pump is actuated at low rate (of the other 1000-1500 r/pm) whereby the disinfecting solution is circulated through the pump, the pipes and the fittings for a time of the order of 2-3 minutes which has been found sufficient for a satisfactory disinfection.

Upon this phase is terminated, by opening the discharge valve 22 the disinfecting solution is removed from the aforesaid circuit.

Then the pipes are rinsed with clean water, with which in the meanwhile the tank 44 had been filled again, repeating the same phases up to discharging again the rinsing water through the line 20.

The plant is now ready for another hydromassage cycle.

If the tank 24 has a volume of the order of 5-6 litres and than the unit dose of disinfecting agent is 0.12 litres the global time for a whole disinfection cycle of the order of 10-15 minutes , by far less than that necessary for the filling of the bath tub and its complete emptying, this operations having also to be repeated for the rinsing with exceeding high consumption of clean water and disinfecting agent.

In the case of the present invention and in the illustrated example by means of 0.12 litres of disinfecting agent a solution is obtained having a concentration of about 2% and with a tank 28 of 3 litres capacity about 25 disinfecting cycle are ensured.

Equivalent variation of the above described plant are possible and foreseable.

For example instead of the reserve tank 28 a distributor can be provided, for example a type used in the standard washing machine.

Moreover the discharge line 20 may be connected for example to a normal shower of which is possibly provided the bath tub, whereby the solution being discharged may be used for the disinfection also of the bath tub.

It is lastly to be noted that the present invention is based on an approach totally different with respect to the prior art, namely to carry out with a closed circulation the disinfection of the only components which are not excessible of the plant, the bath tub or the like being cleaning and disinfected by the conventional method.

For the disinfection and the cleaning of hydromassage installations the components are filled with disinfecting solution, apart from the bath tub, the solution being circulated under reduced pressure and with the delivery fittings in the closed condition, the rinsing being thereafter carried out in the same manner.

**Claims**

1. A process for the disinfection and cleaning of sanitary installation, particularly of hydromassage tub , characterized by the steps of:

(i) filling of the pipes of the circulation, and of the fittings with a disinfecting aqueous solution;

(ii) operating of the circulation pump at a reduced rate and consequently at a reduced hydraulic pressure in order to circulated said solution for the time necessary to carry out the disinfection of said components of the plant filled in the step (i);

(iii) discharging of the disinfecting solution;

(iv) repeating the steps (i) , (ii), and (iii) with rinsing water.

2. A process according to claim 1, characterized in that said operation of the pump is carried out at a rate such that the hydraulic pressure generated is less than the pressure necessary for the opening of the fittings for the jet delivery.

3. Disinfection and cleaning plant for sanitary installation, particularly for hydromassage installations, characterized by comprising an assembly for the feeding of disinfecting aqueous solution having a predetermined concentration or of water in an amount such that, the delivery fittings being closed, all the components of the sanitary installation are filled apart from the bath tub or the like, and means for the control of the operation cycle of the circulation pump of the sanitary installation.

4. Disinfection and cleaning plant according to claim 3, characterized in that said feeding assembly comprises a mixing tank, means for the controlled supply of clean water to said tank, means for the controlled supply of disinfecting agent to said tank and means for the controlled discharge for said tank to the delivery pipe of the circulation pump.

5. Disinfection and cleaning plant according to claim 4, characterized in that said mixing tank is provided with a control valve for the amount of liquid, namely water or disinfecting solution.

6. Disinfection and cleaning plant according to claim 3, characterized in that said control means are adapted to adjust the operation rate of the circulation pump so as to be less than a predermined value.

7. Disinfection and cleaning plant according to claim 3, characterized in that said circulation pump is provided with a pipe for the controlled discharged of the liquid filling the components of the installation.

Fig.1